# EUROPEAN PATENT SPECIFICATION

(11) **EP 2 198 856 B1**
(45) Date of publication and mention of the grant of the patent: **28.02.2018**
(21) Application number: 10158751.7
(22) Date of filing: 30.05.2002
(51) Int. Cl.: A61K 9/16, B01J 2/20, B29C 47/40

(54) **Process for preparing granular compositions**
Verfahren zur herstellung körnigen zusammensetzungen
Procédés pour la preparation des compositions granulaires

(30) Priority: 07.06.2001 GB 0113841
(43) Date of publication of application: 23.06.2010
(62) Divisional of application: 02727770.6
(73) Proprietor: Reckitt Benckiser Healthcare (UK) Limited, Slough, Berkshire SL1 3UH (GB)
(72) Inventor: Dring, Richard James, Nottingham, Nottinghamshire NG2 3AA (GB); Edinborough, Nicholas, David, Nottingham, Nottinghamshire NG2 3AA (GB); Sherry, Robert, Arthur, Nottingham, Nottinghamshire NG9 6LF (GB)
(74) Representative: O'Brien, Niall James

(56) References cited:
- EP-A1- 0 665 009
- WO-A1-97/18839
- WO-A1-02/098387
- WO-A2-01/41733
- DE-A1- 19 809 242
- DE-A1- 19 841 244
- US-A- 4 578 455
- US-A- 5 667 807
- US-A- 5 965 161
- US-A- 6 150 424

## Description

This invention relates to a process to prepare a non-steroidal anti-inflammatory drug composition and to uses thereof.

Non-steroidat anti-infiammatory drugs (NSAIDs) are a widely used class of medicaments. They are a well defined group of compounds and include phenylpropionic acids such as ibuprofen, naproxen, ketoprofen and flurbiprofen. They are primarily used for the treatment of one or more of pain, inflammation and fever, for example rheumatoid arthritis, ankylosing spondylitis, osteoarthritis, postoperative pain, post-partum pain and soft tissue injuries. One example is ibuprofen, which is available under prescription in the UK (eg Brufen (RIM)), generally at doses up to 3200 mg per day. Ibuprofen is also available as a non-prescription drug in the UK (eg Nurofen (RTM)) primarily for the treatment of symptoms of pain and fever including headache, migraine, rheumatic pain, muscular pain, backache, neuralgia, dysmenorrhoea, dental pain and colds and flu, generally at doses up to 1200 mg per day.

It is desired to provide a pharmaceutical composition comprising a low-melting NSAID which is stable, may be provided by an economic process and which has advantageous formulation properties.

European Patent Application No. 362728 (1990) relates to a procedure for obtaining ibuprofen powder that flows easily and has improved storage and formulation properties for direct tabletting. It is said that hardening molten ibuprofen is extremely problematic as the molten ibuprofen that congeals at about 74.8°C becomes oily and viscous as it cools, tends to be under-cool and can be transformed into a crystalline state only very slowly. In accordance with EP 362728, it is proposed that ibuprofen particles are obtained through the solidification of molten ibuprofen on a contact cooler (e.g. a roller or cooling belt) at 0-50°C, using seeding, followed by crushing, It is said that seeding can be carried out by coating with a molten layer that congeals on the contact cooler. The seed can also be added to the roller in particle form or worked into the molten product. It is said that any process to prepare molten ibuprofen can be used to manufacture the ibuprofen particles, although, the procedure is of particular relevance for a molten ibuprofen product obtained through the rectification of ibuprofen in accordance with German Patent Application No 3802619. The single illustrative Example shows that the ibuprofen powder produced by the process requires to be mixed with a significant number of additional excipients prior to tabletting.

It has also been proposed to combine NSAIDs with thermoplastic polymers and extrude the plastic mass. For example, EP 580860 (1994) describes a process for producing a solid dispersion of any drug dissolved or dispersed in a polymer by employing a twin-screw extruder equipped with paddle means. The extrudate is comminuted using an appropriate mill. The twin-screw extruder is said to provide improved processing advantages over a single-screw extruder. WO 9906038 relates to a fast-acting analgesic preparation comprising ibuprofen in an adjuvant matrix, where the preparation has a porous structure and a density of greater than 1 and up to 2.5 g/cm³. The adjuvant matrix comprises water-soluble polymeric binders, carbonates and appropriate pharmaceutical adjuvants. Mixing of the components preferably takes place in an extruder. After extrusion through the extruder outlet, the plastic composition is shaped to a suitable drug form eg by passing the plastic extrudate between two rolls which are driven in opposite directions and have mutually facing depressions in the surface of the rolls.

The presence of a thermoplastic material is not desired in a pharmaceutical composition according to the present invention.

We have now found an advantageous process to prepare a pharmaceutical composition in which the NSAID is melt-extruded and the extrudate is formed into two or more ribbons which are cooled to form solidified melt granules having a low melting NSAID as a continuous phase.

European Patent Application 686392 (1995) relates to a thermal process for the production of directly tablettable granules comprising melt-extruding an active compound having a low melting point and necessary tablet auxiliaries at elevated temperature to give a homogeneous non-agglutinating extrudate which is then comminuted to give tablettable granules. By means of the mixing and kneading elements of the extruder, the mixture is compacted to give an extrudate at a temperature at which a part of the active compound is melted. The extrudate is pressed through a perforated plate to give thin strands of 0.3-2.0 mm diameter and comminuted after cooling to the desired particle size of the granules. The granules thus obtained can immediately be subjected to tabletting, only a lubricant being required. The active is thus present as unmelted crystals within a solidified melt of the active. It is said that by means of the process, all auxillarles such as binder, disintegration auxiliaries, fillers and other auxiliaries can be incorporated directly in the granules.

However, it has now been found that by heating the NSAID to a temperature above its melting point, further processing advantages and formulation advantages are obtained. In particular, the rapid cooling of a thin ribbon of fully molten NSAID increases the production rate. This was not expected, as heating the NSAID to a higher temperature would be expected to require a greater cooling effect, thus reducing the efficiency of the production process, In addition, it has been found that the dissolution rate of the granules prepared from the solid composition have improved dissolution in comparison with compositions wherein the NSAID is present in two crystalline forms.

In co-pending PCT application PCT/EP00/12193 there is described a process to prepare a compressed tablet composition comprising a non-steroidal anti-inflammatory drug having a melting point in the range 30-300°C characterised by:
(a) combining said drug in molten form with a disintegrant to form a uniform mixture;
(b) cooling said mixture to form a solidified melt;
(c) forming said solidified melt into granules; and
(d) compressing said granules, optionally with an extra-granular component, to form a compressed tablet composition.

One of the illustrative methods therein describes melt-extruding a mixture of ibuprofen and croscarmellose sodium (a disintegrant) with heating until the NSAID was fully molten, discharging a continuous molten ribbon of extrudate onto a cooled steel band, cooling and milling the solidified mass followed by mixing with pharmaceutical excipients and compressing into a tablet.

We have now found that if the molten extrudate is formed into a plurality of thin ribbons and cooled rapidly an advantageous production process is thereby achieved.

Accordingly, the present invention provides a process according to Claim 1.

The granular composition may be prepared in accordance with the present invention by a simple cost-efficient manufacturing process on a large scale. Formulations prepared from a composition according to the present invention have been found to be stable on storage and to have advantageous dissolution properties. The formulation may be tabletted without sticking or capping during the tabletting process to provide a dosage form having suitable hardness properties combined with advantageous disintegration properties. Furthermore, the poor taste is significantly improved.

As used herein, "solidified melt granules "means granules formed by meltextruding the non-steroidal anti-inflammatory drug (NSAID) in fully molten form, cooling and forming the mixture into solidified melt granules.

It has been found that the crystalline form of the NSAID changes on melting and then cooling the NSAID For example, the crystals become smaller and the surface area of the NSAID in the melt granule is increased compared to that of conventional crystals of the NSAID. In addition, on cooling, the changes to the crystalline structure lead to a more porous granule.

The crystalline structure of the melt granules formed from solidifying fully melted NSAID differs from the crystalline structure where the NSAID is only partially melted. In the case of partial melting, the crystalline structure of the melted NSAID is interrupted by the non-melted NSAID, thus providing that the NSAID does not have a single crystalline structure. The NSAID is fully melted so that on cooling, a single continuous phase of the NSAID is formed. That is to say the crystalline structure of the NSAID is not interrupted by another crystalline NSAID structure. The melt granules are thus in the form of a solidified melt of NSAID, comprising said NSAID solely as a continuous phase.

The invention allows the formulation of a low melting NSAID into an acceptably tasting, readily disintegrating composition. It is generally envisaged that the melting point of such compounds will be low enough to allow the melting thereof using standard equipment. It is also important that there is not a deleterious effect on the NSAID itself or any ingredients incorporated in the molten NSAID, for example a disintegrant.

The NSAIDs have lower melting points so that the melting process does not use significant amounts of energy, which thus reduces production costs. A favoured class of compounds are the 2-arylpropionic acids which are generally substantially insoluble and have poor taste properties. The NSAIDS have melting points in the range 30-200°C (such as racemic naproxen, melting point 156°C), more preferably 30-150°C, further preferably 40-120°C (such as racemic flurbiprofen, melting point 114°C), most preferably 40-100°C (such as racemic ibuprofen (melting point 75-77°C), S (+)-ibuprofen (melting point 52-54°C) and racemic ketoprofen (melting point 96°C)). The low-melting NSAIDs are naproxen, ketoprofen, flurbiprofen, ibuprofen. Preferably the NSAID is in the form of a racemic mixture or an enantiomer (especially the S (+)-enantiomers) thereof. Salts of racemic NSAIDS and enantiomers thereof may also be used. Preferred salts are the sodium salts, potassium salts and the lysine salts.

The invention is especially adapted for an ibuprofen medicament. The term "ibuprofen medicament"preferably includes racemic ibuprofen and S (+)-ibuprofen and their sodium, potassium and lysine salts which have low melting points and a very poor after-taste in the mouth and throat. Most advantageous results are obtained with racemic ibuprofen which has a high dosage combined with poor solubility properties.

In step (a) the NSAID is melted and extruded. Under conditions of pressure, the drug may be melted at a temperature below its normal melting point. The maximum temperature is determined by the stability of the molten drug and ingredients combined therewith. The drug may be heated to any convenient temperature. Generally, the higher the temperature, the more quickly the drug will melt although this must be balanced by the energy input required to heat the drug. For highest efficiency, the NSAID will be heated to 5-20°C, above its melting point to keep energy costs to a minimum.

The NSAID is generally melted in a heated extruder barrel having an inlet for the solid drug and an outlet for the molten extrudate. The barrel may be divided into different heating zones as desired, In addition, the work on the NSAID by the screw configuration in the extruder will also contribute to melting the NSAID, thereby reducing its external applied temperature requirement. Accordingly the extruder barrel may be heated to a temperature less than the melting point of the NSAID. For example, the normal melting point of racemic ibuprofen is 75-77°C, however under conditions of force/pressure (such as may be encountered in an extruder or similar processing device), the external applied heat necessary to melt the ibuprofen may be reduced significantly through the mechanical heat generated by the intense mixing action within the extruder. It is generally envisaged that the extruder will be heated to a temperature not less than 25°C below the melting point of the drug, preferably in the range from 20°C below the melting point of the drug to 50°C above the melting point of the drug, more preferably from 15°C below the melting point of the drug to 25°C above its melting point and most preferably to a temperature in the range of 10°C on each side of the melting point of the drug. Some extruders allow different zones to be heated to different temperatures in the extruder. These temperatures can be chosen as desired to ensure that the NSAID is fully melted in step (a). Preferably, the drug and optional excipients, for example a disintegrant, are heated to a temperature in the range 80-13G°C, more preferably 100-120°C to melt said drug. When the NSAID is ibuprofen it may conveniently be heated in the range 50-130°C, more preferably 60-100°C. The temperature of the ibuprofen in the extruder barrel is preferably in the range 66-96°C, preferably 70-82°C.

The extruder may also have one or more cooling zones. The cooling zones may be necessary to remove the heat generated by the kneading action on the material being extruded, particularly to ensure that there is a good flow of material into the extruder and out from the extruder.

In a preferred process according to the present invention, the extruder is provided with a cooling zone and a heating zone. Further preferably, there is provided a cooling zone at the inlet portion of the extruder so that the material entering the extruder may be conveyed or transferred along the extruder to a heated zone. In the cooling zone, the internal heat generated within the material being extruded is carried away so that partial melting of the NSAID cannot occur which may be detrimental to the throughput of material in the extruder. Preferably, the extruder is provided with a cooled transfer zone and a heated melting zone.

In a further preferred process, there is provided a heated zone at an end portion of the extruder at or adjacent the outlet. The extruded material may be heated to ensure that the extrudate passing through the extruder outlet is sufficiently heated so that the temperature difference between the molten extrudate and extrudate cooling means is maximised as appropriate to optimise the cooling process. For example, the barrel may be heated to cause the extrudate passing through the outlet to be preferably fully molten or substantially fully molten. The pressure within the extruder may cause a lowering of the melting point of the NSAID. Accordingly, preferably, the temperature of the extrudate passing through the outlet is in the range of 20°C on each side of the normal melting point of the drug, preferably within 10°C on each side of the melting point of the drug.

The extruder is suitably provided with at least one screw shaft provided with means arranged to generate heat within the NSAID. This may usually be achieved by a combination of kneading paddles and helical screws. Generally, it is preferred to provide helical screws at the inlet portion to convey the material away from the inlet. The material may be extruded in the extruder barrel with screws and/or with paddles. It is preferred to use more than one screw shaft, for example a twin-screw shaft, to maximise the extrusion effect on the material being extruded. The use of paddles also maximises the shear effect on the material being extruded. The paddles may be offset at any desired angle or combination of angles to generate internal heat within the drug as appropriate to melt the drug. The configuration and/or size of the paddles will depend on factors such as the diameter and/or length of the extruder, the ratio of the length to the diameter, the extruder speed, the torque applied and the desired temperature to melt the NSAID.

The screws and/or the paddles may be in the forward and/or reverse direction to maximise the pressure within the mixing zone as desired.

A preferred arrangement comprises helical transfer screws at inlet portion of the extruder, a plurality of paddles which may have differing sizes and degrees to which they are offset and further helical transfer screws at the outlet portion to convey the extrudate out of the extruder. Further preferably the helical transfer screws at the outlet portion may comprise a reverse helix followed by a forward helix.

A preferred feature of step (a) is that a disintegrant is also combined with said drug in molten form. In a further preferred feature of step (a), at least one of a surfactant and a diluent is combined with said drug in molten form.

Conveniently, the NSAID may be de-aerated as it is transferred to the melting zone.

In a further process, the non-steroidal anti-inflammatory drug may be combined with optional excipients, eg a diluent, and then heated together until said non-steroidal anti-inflammatory drug is molten. In yet a further process the NSAID and optional excipients may be combined in the solid state and extruded together until said NSAID is molten and any further desired excipients uniformly blended with the mixture.

Alternatively, the NSAID may be melted before it is combined with any optional excipients.

In step (b), a homogeneous extrudate is formed which passes out through the outlet of the extruder. Preferably, the NSAID is fully molten as it exits the extruder. The extrudate may consist of said NSAID, without additional ingredients, wherein the NSAID is present as a single continuous phase. Optionally, the extrudate may contain additional excipients, for example one or more of a disintegrant, a surfactant and a diluent, which are blended within the molten NSAID.

In step (b), the extrudate is formed into two or more thin ribbons. This is preferably achieved by passing the molten extrudate through channels at the outlet which form streams or ribbons of extrudate which may be directed onto the cooling means, preferably a cooling belt or a cooling drum.

The ribbons of molten extrudate are cooled rapidly by said cooling means, ie the ribbons solidify in 5 minutes or less, preferably in 3 minutes or less, more preferably in 1 minute or less (eg 0-60 seconds), preferably in 50 seconds or less (eg 1-50 seconds), more preferably 1-40 seconds and most preferably 1-30 seconds.

Suitably, the width of each ribbon of molten extrudate is greater than the depth of the ribbon so that cooling is optimised. The width of each ribbon will. depend, at least to some extent, on the viscosity of the molten extrudate. Each ribbon of molten extrudate has a depth on the cooling means of 10 mm or less, preferably up to 6 mm (for example 0.1-6 mm), preferably 0.5-5 mm, for example 3-4 mm and most preferably 1-3 mm, for example 2 mm.

Cooling will normally occur first on the side of the ribbon proximate the cooling means. Accordingly, usually the lower surface of the ribbons solidifies while the upper surface of the ribbon is still molten. As the ribbon is further cooled, the extrudate solidifies throughout its depth.

To maximise output, a plurality of ribbons are provided extending parallel to each other, for example on a cooling belt. Preferably, there are more than two ribbons, for example three, four, five, six, seven, eight, nine or ten or more ribbons according to the size of the extruder. The number of ribbons may be limited by the width of the ribbon formed and the whole width of the cooling means which provides for a maximum number of ribbons. It has been found that the ribbons of
molten NSAID do not spread on the cooling means, accordingly there requires only a small space between the ribbons.

As hereinabove discussed, it is preferred to have a significant temperature difference between the molten extrudate and the cooling means as the extrudate comes into contact with the cooling means, for example at least 25°C, preferably at least 35°C, more preferably at least 45°C and most preferably at least 55°C. The upper end of the above ranges is limited by the melting point of the drug, but it is not desired to heat the extruded material to too high a temperature as the extra energy costs will not be balanced by any processing advantage. Accordingly, a practical upper limit to each of the above ranges is 100°C, more usually 80°C.

Generally, it is expected that the molten mixture will be cooled to a temperature below the melting point of the drug before being formed into granules. The molten NSAID may be poured onto cooling trays which may be static or continuously moving. Static trays may be placed in cooling cabinets. Moving trays or belts may have additional cooling means, such as cooled water. The cooled melt forms a solid and may be scraped off the belt or collected as it falls off one end of a continuously moving belt Preferably the molten drug mixture may be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten drug mixture and the speed of the belt, It is especially preferred to cool the molten drug mixture at least initially by cooling means, for example until it has started to solidify. Advantageously, the belt is water-cooled along substantially the whole of its length and it is of minimum length required (e.g. 3-7m) to allow it to cool to the solid state.

The solidified melt may be formed into granules by a plurality of methods. For example, it may be pulverised or comminuted into granules, It may be milled and/or sieved. If it is cooled on a moving belt or drum, the cooled melt may be broken into conveniently sized pieces, followed by milling and or sieving.

The granular composition may be sieved to ensure that the melt granules are of the appropriate size for efficient tabletting. The granules produced on cooling the molten drug are preferably of a suitable size for tabletting, preferably in a standard large scale tabletting machine. The melt granules in the granular composition preferably have a mean particle size in the range 10-2000µm, more preferably 50-1000 µm and most preferably 100-400 µm. Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6gml⁻¹). Further, preferably the melt granules have a porosity of 0.5-2.0 g/ml.

The proportion of NSAID in the melt granules will depend on the dose desired for therapeutic effect. Low dose drugs, such as flurbiprofen and ketoprofen may form as little as 1% by weight if a relatively large dosage form is required. However, particular advantages of the present invention are obtained by allowing a reduction in the number and/or amount of excipients. Accordingly the NSAID may form up to 100% w/w of the melt granules. A feature of the invention is that low-melting, high dose NSAID. Ds, such as ibuprofen, can be formulated with a disintegrant into smaller dosage forms. Accordingly, the NSAID will suitably form 70-95% w/w and preferably 80-95% w/w of the melt granules.

The solidified melt granules may be formulated directly or they may be combined with an extra-granular composition and formulated into a unit dose. The melt granules are combined thoroughly with extra-granular composition so as to form a uniform mixture of ingredients. This may be achieved by conventional mixing and blending techniques. Examples of apparatus that may be used to facilitate this process are. Ribbon Blender, IBC Blender, V-Bender and Plough Benders. Examples include filling of the loose powder mixture into a sachet or a capsule or
compressing it into a tablet. Tablets are the preferred unit dosage form according to the invention. They may be swallowed or they may be chewed. It has unexpectedly been found that the taste of the NSAID has been substantially masked which allows the dosage form to be maintained in the oral cavity for a period of time whilst the formulation is swallowed.

The compressed tablet composition of the present invention may optionally be coated with a film coat, for example based on a conventional cellulose polymer such as hydroxypropylmethylcellulose, or a conventional sugar coat, for example based on sucrose or lactose.

The granular composition also comprises a disintegrant. The addition of a disintegrant further improves the rate at which the tablet breaks up when added to a liquid medium. Disintegrants are those which swell on the action of water, thus causing the ingredients in the tablet to be pushed apart and out into the aqueous disintegration medium. Disintegrants are croscarmellose sodium and/or sodium starch glycolate, especially croscarmellose sodium. The disintegrant is present at an effective disintegrating amount, for example up to 50% w/w of the formulation (eg 1-50% w/w), more preferably 1-25% w/w, further preferably 2-20% w/w and most preferably 2-15% w/w of the formulation.

The disintegrant is an ingredient of the melt granules and/or the extragranular composition. The disintegrant will suitably form 0.1-25% w/w of the melt granules, preferably 3-15% w/w and most preferably 4-10% w/w of the melt granules. The disintegrant may be present in the extra-granular composition in an amount of 0.1 to 25% w/w, preferably 1-15%, more preferably 2-10% w/w.

The ratio of NSAID to disintegrant will depend on the proportion of the NSAID in the dosage form. Thus, depending on the dosage of the drug, it can be expected to fall in the range 20:1 to 1:20, conveniently 10:1 to 1:10. For relatively high dose drugs, such as ibuprofen and naproxen, the ratio of NSAID to disintegrant may be in the range 20:1 to 2:1, preferably 10:1 to 5:1 parts by weight. For relatively low dose drugs, such as flurbiprofen and ketoprofen, the ratio of NSAID to disintegrant is suitably 1:10 to 10:1, preferably 1:1 to 1:5 parts by weight.

Although not necessary for the production of compositions according to the present invention, if desired, the dosage form may further comprise a diluent. The diluent, may be water-soluble or water-insoluble. Suitable water-soluble diluent materials include the sugar alcohols (such as xylitol, sorbitol, mannitol, erythritol), sugars (such as sucrose, fructose, lactose, dextrose), cyclodextrin, maltodextrin and salts of organic acids (eg sodium citrate and potassium citrate). Lactose, sodium citrate and potassium citrate are particularly preferred water-soluble diluents. Suitable water-insoluble diluent materials include cellulose derivatives (such as microcrystalline cellulose) starch and derivatives thereof (such as pre-gelatinised starch), dicalcium phosphate, tricalcium phosphate, calcium sulphate, calcium carbonate. Macrocrystalline cellulose and dicalcium phosphate are preferred water insoluble diluents.

The diluent may preferably include a basic ingredient such as an alkali metal salt for example an alkali metal carbonate, bicarbonate or citrate, present to an extent of up to 50% by weight (eg in the range 1-50% by weight), preferably up to 40% by weight (eg in the range 1-40% by weight) of the formulation (more preferably 2-35% w/w and most preferably 10-20% w/w). Preferably, the alkali metal salt is sodium or potassium. Further preferably, the salt is a citrate, carbonate or bicarbonate salt of sodium or potassium, more preferably sodium bicarbonate or citrate. The ratio of NSAID (especially ibuprofen medicament) to alkali metal salt may be in the range 100:1 to 1:1 parts by weight, preferably 5:1 to 1:1 parts by weight. Preferably, the alkali metal salt is incorporated in any amount up to an equimolar amount with respect to the NSAID (eg ibuprofen). Conveniently, a sub-molar amount of alkali metal salt is incorporated. Thus the alkali metal compound may form up to 100% w/w of the NSAID, preferably 50% w/w, more preferably up to 10% w/w, of the NSAID. In a preferred compressed tablet according to the present invention, the NSAID (especially an ibuprofen medicament) is in admixture with the alkali metal salt. The alkali metal salt is preferably incorporated in the melt granules.

In a formulation adapted to disperse in water prior to administration, the level of diluent may be quite high, for example up to 50% (such as 0-50% w/w, preferably 0-40% w/w) by weight of the formulation in order to achieve the desired dispersing properties. Preferably, the diluent does not form greater than 25% by weight of the formulation (eg 0-25% w/w), as it adds to the costs of the composition and to production costs. Thus, to minimise costs it may be preferred that the diluent is added to the formulation in an amount of 0-20% by weight of the formulation, more preferably 0-10% w/w. If present, it may be preferably used to an extent of 0.1-25% by weight of the formulation, more preferably 0.1-20% w/w, further preferably 0,1-10% w/w and most preferably 1-5% by weight of the formulation.

The diluent may be contained in the melt granule or may be part of the extra-granular composition or may be incorporated as desired in both components. If desired, the diluent may preferably be added in an amount up to 30% w/w of the extra-granular component (ie 0.1-30% w/w, although to minimise the size and cost of the dosage form, it is desired to include a minimum amount of such additional excipients. Accordingly, if employed, the diluent may suitably be included in the extra-granular composition in the range up to 30% w/w (ie 0.1-30%), preferably 0.1-15% w/w, more preferably 0,1-10% w/w. As discussed hereinabove, the diluent may be present in the melt granules, for example in an amount of 0-30% w/w (such as 0,1-30%) by weight of the melt granules. If present, the diluent conveniently forms 1-20%, more preferably 1-10% w/w of the melt granules.

The formulation may also include a surfactant, in the amount appropriate to the properties of the surfactant, preferably 0.05-10% by weight of the formulation. The surfactant may be included in the melt granules and/or the extra-granular composition. Preferred surfactants are sodium lauryl sulphate, poloxamer, hydrogenated castor oil and derivatives thereof, polyoxyethylene surfactants (including polyoxyethylene oils, fatty acid esters, including stearates) and sorbitan esters. They may be used to an extent of 0.05-5% w/w, preferably 0.1-3% w/w, more preferably 0.2-2% w/w) of either or both the melt granules and the extra-granular composition.

The extra-granular composition comprises the ingredients incorporated in the formulation which are not contained in the solidified melt granules. The ingredients of the extra-granular composition may be mixed with the melt granules simultaneously or at sequential stages in the process to prepare the formulation. A particular advantage of the present invention is preferably that all the ingredients of the extra-granular composition are combined with the melt granules at a single stage in the manufacturing process. Also, it is preferred that at this stage, the ingredients in the extra-granular composition are combined sequentially with the melt granules. The formulation comprises a uniform mixture of melt granules and extra-granular composition. The extra-granular composition is suitably distributed evenly throughout the formulation.

The extra-granular composition may also comprise flow acids such as colloidal silicon dioxide and talc. The colloidal silicon dioxide is insoluble and suitably has a surface area greater than 50 m²g⁻¹, more preferably greater than 100 m²g^{-1,}, especially in the range 150-250 m²g⁻¹.

Optionally a lubricant may be incorporated in the extra-granular composition for mixing with the melt granules. Conventional lubricants for NSAIDs may be used for example stearic acid, sodium lauryl sulphate, polyethylene glycol, hydrogenated vegetable oil, sodium stearyl fumarate, magnesium stearate or calcium stearate. These may be present in an amount from 0.05-5% by weight, preferably 0,1-2% by weight of the formulation. Anti-adherents such as talc, may further be included in an amount of up to 4% by weight of the dosage form, for example 0.5-2% by weight of the dosage form, preferably as part of the extra-granular component.

Other conventional tabletting excipients known to the person skilled in the art may be incorporated in the compressed tablet composition according to the present invention as desired, although it will be appreciated that a prime advantage of the present invention is that the number of excipients necessary to achieve a quickly disintegrating tablet with good dissolution characteristics is minimal.

A disintegrant is preferably the sole ingredient incorporated within the NSAID (preferably ibuprofen) melt granules or it may be combined with a diluent and optionally a surfactant and other tabletting excipients. Accordingly, in one preferred embodiment, the granules may comprise greater than 90% w/w of the NSAID and disintegrant (ie 90-100% w/w). Preferred melt granules comprise an NSAID (preferably ibuprofen), a disintegrant and optionally a surfactant and/or a diluent. In a further preferred embodiment, the formulation comprises greater than 90% w/w (ie 95-100% w/w) of the combination of NSAID, acidic component and disintegrant. Further preferably, the formulation consists essentially of (i.e. 98-100% w/w) of the combination of NSAID and disintegrant. Further preferred melt granules consist essentially of an NSAID (preferably ibuprofen), a disintegrant and a surfactant. Further preferred granules consist essentially of an NSAID (preferably ibuprofen), a disintegrant and a surfactant. Preferably the NSAID is ibuprofen.

The compressed tablet composition of the present invention may, if desired, include other compatible pharmacologically active ingredients and/or enhancing agents. Thus, for example, the dosage form may include any other ingredient commonly used in a composition useful to treat pain, inflammation and/or fever, for example caffeine or another xanthine derivative, another analgesic, for example codeine, a skeletal muscle relaxant; an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)) ; a decongestant (eg pseudoephedrine, phenylpropanolamine and phenylephrine) ; a cough suppressant (eg caramiphen, codeine or dextromethorpan); and/or an expectorant (eg guaifenesin, potassium citrate, potassium gualacolsuphonate, potassium sulphate and terpin hydrate).

Such extra active ingredients and/or enhancing agents may be incorporated in the melt granules or in the extra-granular composition in appropriate dosage amounts for the desired therapeutic effect. Reference may be made to MIMS and the Physicians Desk Reference for guidelines as to a suitable dosage. It is generally expected that such other active ingredients will form 0-50% w/w of the formulation, for example 5-25% w/w.

Ibuprofen and its derivatives are primarily anti-inflammatory, analgesic and antipyretic agents but have also been proposed for other therapeutic uses, including the treatment of periodontal bone loss, pruritus and Alzheimer's disease. The dosage forms of the present invention are therefore indicated for use in the treatment of all therapeutic uses for which ibuprofen is effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following:respiratory infections, colds or influenza, gout or morning stiffness.

Accordingly, in another aspect of the present disclosure there is provided a composition according to the present disclosure for use in the treatment of pain and/or inflammation and/or fever. Furthermore, the disclosure also provides a method of treating pain and/or inflammation and/or fever comprising the
administration of a composition according to the present disclosure to a mammal in need thereof.

Unit dosages for effective therapy are known to those skilled in the art for each NSAID. For example, they may comprise the NSAID to an extent of 5mg, 10mg, 12.5mg, 25mg, 50mg, 100mg, 150mg, 200mg, 250mg, 300mg, 350mg, 400mg, 500mg, 600mg and 800mg. Where derivatives are employed, normally the precise unit dosages are chosen to give the equivalent NSAID doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit dose may be 25 mg. Preferred unit doses are in the range 25-100 mg, more preferably 25-75 mg and especially 50 mg ketoprofen. In a preferred feature of step (a), the extra-granular composition further comprises at least one of silicon dioxide, stearic acid or a salt thereof, a surfactant and diluent.

In a preferred process according to the present invention, said NSAID comprises ibuprofen.

The invention is illustrated by the following non-limiting Examples. In the Examples, the racemic ibuprofen is available from BASF Pharma, USA; colloidal silicon dioxide (also known as colloidal silica) is available from Degussa, Frankfurt, DE under the trade name Aerosil 200; and Croscarmellose sodium is available from the FMC Corporation Brussels, BE under the tradename Ac-Di-Sol.

### Dissolution Measurement

The dissolution may measured using the dissolution method described in the US Pharmacopoeia Vol. 23, page 1791, Apparatus 2 using paddles at 50 rpm and a phosphate buffer (selected at pH 7,2 and/or pH 6.0 and/or pH 5.8).

### Friability Measurement

This test for the robustness of the tablet is a standard friability test, namely the rotation of 20 tablets for 4 minutes at 25rpm in a friabulator (TAR 20 manufactured by ERWEKA). The number of capped or broken tablets may be measured.

### Crushing Strength (N)

The crushing strength is a measure of the hardness of a tablet. It may be measured by recording the diametrical crushing strength when the tablet is broken between the motorised jaws of a Schleuniger crushing strength tester.

### Disintegration Time (Minutes)

The disintegration time is measured using the disintegration method described in the European Pharmacopoela 1986, Ref V.5.1.1 (updated 1995) using tap water (pH approximately 7) as the liquid. The method measures the time (seconds) by which six tablets prepared with each Example formulation disintegrates.

### Figures

Fig 1 represents a side-view of the melt-extrusion apparatus.
Fig 2 represents a perspective view of an extruder with the top portion of the barrel removed showing details of the screw configuration.
Fig 3 represents a plan view of an endplate mounted on the extruder.
Fig 4 represents a perspective-view showing the extrudate being fed from the endplate mounted on the extruder onto the cooling belt.
Fig 5 shows the dissolution results for Samples 1, 5, 12 and 16 of Table 3.
Fig 6 shows the dissolution times for tablets produced using a range of compaction pressures from 45 MPa to a mean pressure of 245 MPa.
Fig 7 provides a comparison of the effect of storage for 6 months at 40°C and 75% relative humidity on the dissolution properties of tablets produced according to the present invention and standard starch containing tablets.

### Extrusion apparatus

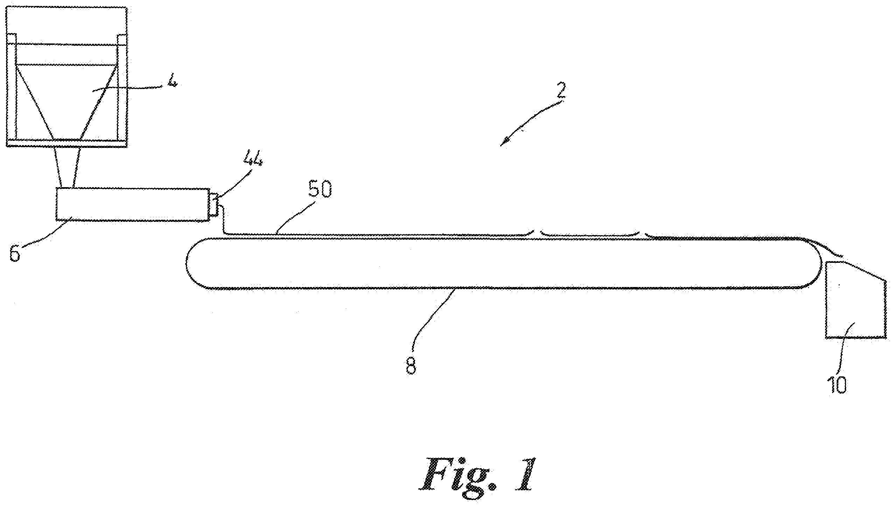

Referring to Figure 1, the extrusion apparatus (2) comprised a feeder hopper (4), an extruder (6) with an endplate (44) mounted thereon, a cooling belt (8) to cool ribbons of extrudate (50) and a collection hopper (10).

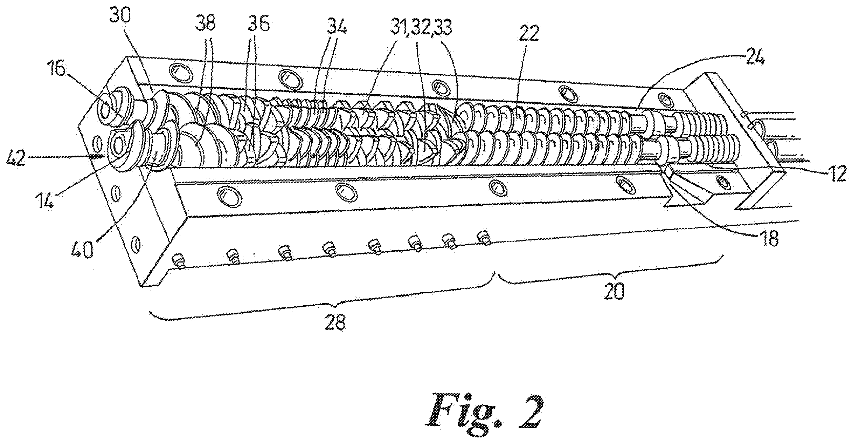

Referring to Figure 2, the extruder was an APV MPC40 twin-screw extruder (having a 40mm diameter barrel; a length: barrel diameter ratio of 1:20 and used at an extruder speed of 600 rpm). The extruder included an extruder barrel (12), twin-screw shafts (14,16), a powder inlet (18), a transfer zone (20) (44cm in length) enclosed by a water cooled jacket (24), a heated mixing zone (28) (38cm in length) enclosed by a thermal jacket (30) and an extruder outlet (42).

In the transfer zone (20), the screw-shafts are each provided with intermeshing forward rotating helical screws (22) by which the NSAID, optionally mixed with excipients, is transferred to the heated mixing zone (28). During the transfer, the NSAID is de-aerated and the heat generated within the NSAID is removed by the water-cooled jacket (24). In the heated mixing zone (28), the screw-shafts are each provided with an arrangement of positive scraping paddles (31,32,33) offset at 30°, 60° and 90° respectively, to knead material in the heated mixing zone, half size paddles (34) to further work the mixture and to generate internal heat within the drug mixture, intermeshing kneading paddles (36) arranged in such a way as to provide a thorough mixing action, reverse helical screws (38) to provide a reverse flow to maintain pressure within the mixing zone and further helical forward rotating screws (40) to carry the molten extrudate through the outlet (42) of the extruder.

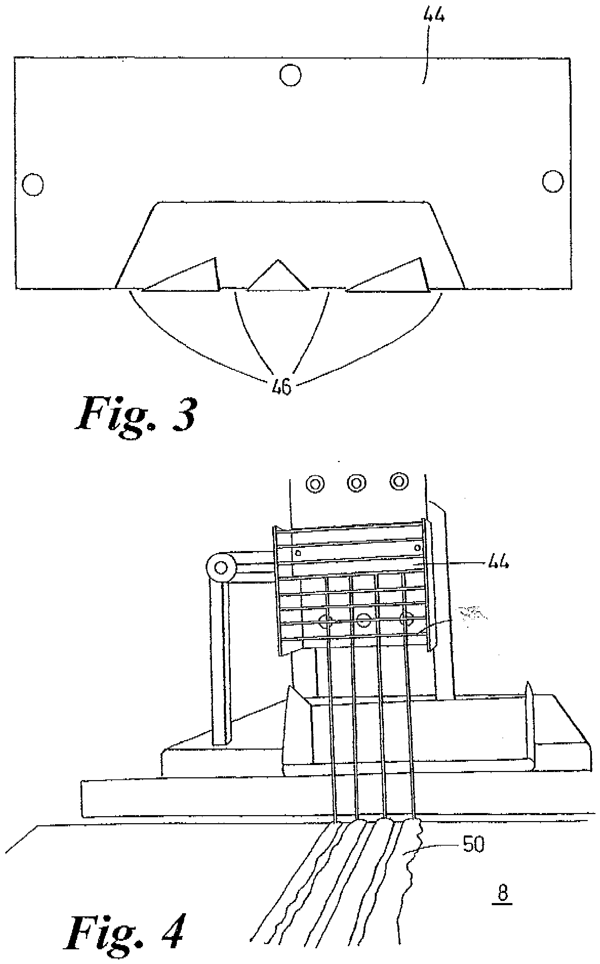

Referring to Figures 1 and 3, the endplate (44) is mounted at the end of the extruder barrel so that the liquid extrudate passes into the endplate from the extruder barrel. The endplate is heated by thermal transfer from the main extruder
block. Channels (46) in the endplate divide the molten extrudate into a plurality of thin streams.

Referring to Figure 4, the streams of molten extrudate flow from the endplate (44) to form ribbons (50) on a continuously rotating stainless steel cooling belt (8) (at a rate of 6.6m/rnin) water-cooled along its entire length. The length of the belt from the endplate (44) to the collection hopper (10) is 4m. A protection grill (46) protects the interruption of the extrudate flowing out of the endplate.

### Example 1

| Ingredients | |
|---|---|
| Ibuprofen | 88.7kg |
| Croscarmellose sodium | 13.3kg |

The ibuprofen & croscarmellose sodium were thoroughly pre-mixed in a tumble blender to form a homogeneous mixture. The solid powdered mixture was fed through the feeder hopper (4) into the powder inlet (18) at a rate of 102 kg/hr into the water-cooled transfer zone (20) of the extruder, In the transfer zone (20), the powdered mixture was thoroughly kneaded between the helical screws and transported to the heated mixing zone of the extruder where the extruder barrel was heated to 100 °C. in the heated mixing zone, the mixture was worked between the intermeshing paddles (31, 32, 33) which generated shear-induced heat to ensure that the ibuprofen was fully molten. The temperature of the extruded material reached approximately 100 - 120°C. The extruded material was then further worked by the half-size paddles (34), the kneading paddles (36) and the reverse helical screws (38) which created a back pressure to the system, further pressurising the heated zone. The extrudate was fed by the forward rotating screws (40) through the outlet of the extruder (42) under pressure and through the endplate (44). The channels (46) in the endplate divided the flow of the molten extrudate into four substantially equal streams.

The four molten streams dropped onto the continuous cooling belt (8), The ribbons formed on the belt were 1-2 cm in width & 2-3 mm thick. The molten streams of extrudate formed four ribbons (50) which cooled rapidly within a period of 30 seconds. The underside of the ribbon in contact with the belt solidified first, followed shortly by solidification of the upper surface of the ribbon. Due to the differential cooling between the upper & lower surfaces of the ribbons, the minor expansion on cooling caused the ribbons to lift upwardly of the belt and break into sections. The sections formed into concave bows. As the upper surface of the melt solidified, the ribbons straightened out to lie on the belt. The lengths of ribbon fell into a hopper at the end of the belt. The belt did not require cleaning as the extrudate lifted cleanly from the belt surface.

The lengths of solidified extrudate were milled & sieved through a screen with a round hole size of 1 mm to provide a granulate material.

### Examples 2-19

The extrusion conditions for the Example 1 composition were varied and are shown in Table 1.

### Examples 2-19:

**EXTRUDER PROCESS CONDITIONS : TABLE 1**

| **Trial Number** | **Barrel Temperature °C** | **Belt Speed m/min** | **Output kg/hr** | **Torque %** | **Extruder Speed rpm** | **Ribbon Thickness/mm** |
|---|---|---|---|---|---|---|
| 2 | 100 | 6.6 | 96 | 20 | 600 | 3.68 |
| 3 | 100 | 6.3 | 103 | 20 | 600 | 4.44 |
| 4 | 105 | 6.6 | 98 | 20 | 600 | 2.79 |
| 5 | 105 | 12.2 | 99 | 20 | 600 | 2.41 |
| 6 | 1 05 | 12.2 | 123 | 25 | 600 | 3.94 |
| 7 | 105 | 6.3 | 87 | 21 | 400 | 2.79 |
| 8 | 110 | 6.6 | 96 | 18 | 600 | 2.54 |
| 9 | 110 | 12.2 | 99 | 18 | 600 | 2.16 |
| 10 | 110 | 12.2 | 112 | 21 | 600 | 2.29 |
| 11 | 110 | 12.2 | 121 | 23 | 600 | 2.54 |
| 12 | 110 | 6.3 | 99 | 21 | 500 | 2.54 |
| 13 | 11 5 | 6.6 | 124 | 23 | 600 | 2.54 |
| 14 | 115 | 12.2 | 116 | 21 | 600 | 1.90 |
| 15 | 115 | 6.6 | 97 | 18 | 600 | 2.41 |
| 16 | 115 | 10.0 | 96 | 18 | 600 | 2.16 |
| 17 | 120 | 6.6 | 96 | 17 | 600 | 2.16 |
| 18 | 120 | 9.9 | 126 | 22 | 600 | 2.16 |
| 19 | 120 | 12.2 | 132 | 24 | 600 | 2.28 |

| | | | | | | |
|---|---|---|---|---|---|---|
| Note: 1. All extrudate samples produced at all of the process conditions were of satisfactory appearance | | | | | | |

### Example 20

Variations may also be made to the extrusion conditions in Example 1 by:
(a) changing the diameter of the extruder barrel, eg to 50 mm;
(b) changing the L:D ratio of the extruder barrel, eg to 17.5:1, 25:1 or 40:1; and
(c) removing and/or adding at least one section of paddles and/or screws.

### Examples 21-23

| | **Ex 21 (% w/w)** | **Ex 22 (% w/w)** | **Ex 23 (%w/w)** |
|---|---|---|---|
| Granular composition: | | | |
| Ibuprofen | 79.1 | 79.1 | 72.2 |
| Croscarmellose sodium | 11.9 | 19.5 | 11.0 |
| Sodium citrate | 7,6 | - | 15.7 |
| Sodium lauryl sulphate | 0.2 | 0.2 | - |

| Extra-granular composition: | | | |
|---|---|---|---|
| Colloidal silicon dioxide | 0.4 | 0.4 | 0.4 |
| Stearic acid | 0.8 | 0.8 | 0.7 |

The ingredients of the granular composition may be melt-extruded and formed into melt granules as described in Example 1. The melt granules were then combined with the ingredients of the extra-granular composition to form a uniform mixture and compressed into tablets containing 200mg ibuprofen.

### Example 24

Extruder Barrel Temperature Profile.

The method set out in Example 1 was used. The extruder screw speed was set to a constant 600 rpm. The powder feed rate was also set to a constant value to give a constant feed rate of powder into the extruder. The method was repeated seven times at different extruder temperatures of from 95 °C to 140 °C, as shown in Table 2. The product of each trial was analysed to determine the level of the two major ibuprofen degradation products: 2-(4-isobutyrylphenyl) propionic acid (BTS 47711) and 4-isobutylacetophenone (BTS 40655).

**Table 2**

| **Control Parameter** | | | **Output/Observation** | | | |
|---|---|---|---|---|---|---|
| **Trial No** | **Temp** | **Pdr feed** | **Output** | **Ibuprofen mg/tab. Equivalent** | **Degradation** | **Products** |
| | **C** | **0-10** | **Kg/hr** | | **BTS 47711** | **BTS 40655** |
| 1 | 95 | 3.0 | 102 | 198.4 | 0.09 | ND |
| 2 | 100 | 3.0 | 102 | 198.8 | 0.09 | ND |
| 3 | 105 | 3.0 | 102 | 198.6 | 0.09 | ND |
| 4 | 110 | 3.0 | 110 | 197.7 | 0.08 | ND |
| 5 | 120 | 3.0 | 114 | 198.8 | 0.09 | ND |
| 6 | 130 | 3.0 | 120 | 198.2 | 0.09 | ND |
| 7 | 140 | 3.0 | 120 | 198.4 | 0.09 | ND |

### Discussion of Results

These results show there was no increase in the impurity 2-(4-isobutyrylphenyl) propionic acid (BTS 47711) over that normally present in the ibuprofen raw material and no detectable level of the degradation product 4-isobutylacetophenone (BTS 40655).

Therefore, under the conditions of the test up to a maximum extruder setting of 140 °C, there was no evidence of ibuprofen degradation as a result of the melt extrusion process.

### Example 25

### Extruder Temperature/Throughput Matrix

Further trials using the method of Example 1 but under process conditions as shown in Table 3 were conducted to determine the maximum output from the extruder by varying barrel temperature and powder feed rates, as shown in Table IIA.10.

Once the required extruder barrel temperature had been reached, the extruder was started and allowed to run for 15 minutes in order to equilibrate prior to sample collection.

**Table 3 Extruder Temperature/Throughput Matrix**

| | | | | | **Degradation** | **Products** |
|---|---|---|---|---|---|---|
| **Sample** | **Temp °C** | **Pdr feed 0-10** | **Output Kg/hr** | **Ibuprofen Mg/tab** | **BTS 47711** | **BTS 40655** |
| 1 | 95 | 3.0 | 102 | 198.4 | 0.09 | ND |
| 2 | 100 | 3.0 | 102 | 198.8 | 0.09 | ND |
| 3 | 105 | 3.0 | 102 | 198.6 | 0.09 | ND |
| 4 | 110 | 3.3 | 126 | 198.1 | 0.09 | ND |
| 5 | 110 | 3.6 | 138 | 199.0 | 0.09 | ND |
| 6 | 110 | 3.8 | 156 | 198.2 | 0.09 | ND |
| 7 | 110 | 4.2 | 182 | 198.7 | 0.09 | ND |
| 8 | 115 | 3.5 | 138 | 198.3 | 0.09 | ND |
| 9 | 115 | 4.0 | 156 | 198.7 | 0.09 | ND |
| 10 | 115 | 4.5 | 174 | 198.7 | 0.09 | ND |
| 11 | 120 | 3.5 | 138 | 198.9 | 0.09 | ND |
| 12 | 120 | 4.0 | 156 | 198.7 | 0.09 | ND |
| 13 | 120 | 4.5 | 180 | 194.6 | 0.09 | ND |
| 14 | 120 | 5.0 | 195 | 199.2 | 0.09 | ND |
| 15 | 130 | 5.0 | 186 | - | - | - |
| 16 | 130 | 6.0 | 216 | 196.7 | 0.09 | ND |

Samples were collected for analysis to determine the level of degradation products formed.

In addition, the extrudate of samples 1, 5, 12 and 16 of Table 3 was milled to form granule. Colloidal silicon dioxide (1 mg/tab) and stearic acid (2 mg/tab) were added as process aids and the granules compressed into tablet cores. These cores were tested for dissolution performance using the standard USPII 50 rpm paddle dissolution method as described above.

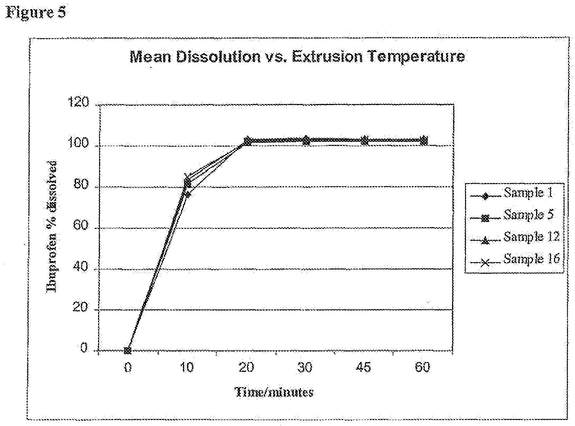

The results are presented in Figure 5.

### Discussion of Results

Samples were obtained using extrusion temperatures of from 95 °C to 130 °C at production output rates of from 102 kg/hour to 216 kg/hour. Under these, there was no evidence of degradation of ibuprofen over the range of extruder barrel temperatures and throughputs investigated.

The dissolution performance of the samples tested was found to be independent of the production throughput within the ranges tested.

### Example 26

### Tablet dissolution vs. Compaction Pressure

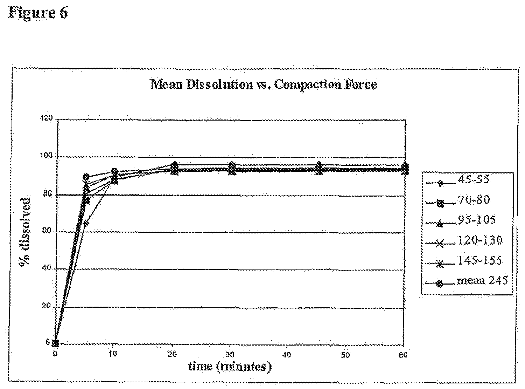

The dissolution time of tablets produced using a range of compaction pressures ranging from 45 MPa to 210 MPa was measured using the dissolution test method described above. The results are presented in Figure 6.

### Discussion of Results

The results show that the dissolution profiles of the tablets produced over a wide range of compaction pressures of from 45 MPa to 240 MPa are almost identical, This demonstrates that the formulation is not sensitive to the tablet compaction pressure applied during production.

### Example 27

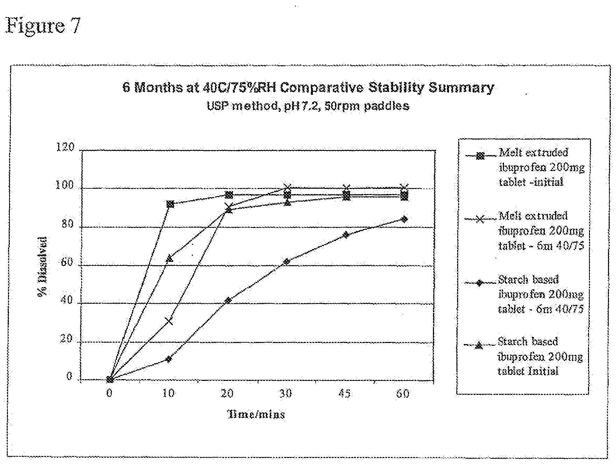

A comparative stability study was carried out. The stability of ibuprofen containing tablets initially and after 6 months at 40°C and 75% relative humidity was compared with that of ibuprofen tablets containing standard starch based granules. This study was carried out using the dissolution test method described above. The results are shown in Figure 7.

### Discussion of Results

The results show that the dissolution properties of tablets produced using the process of the invention were not affected by storage conditions used, while the dissolution properties of the standard starch based tablets were adversely affected by these storage conditions.

### Example 28

The method of Example 1 was repeated, except that 88.7 kg Flurbiprofen was used instead of Ibuprofen and extruder barrel of the heated mixing zone was heated to 130°C to ensure the flurbiprofen was fully molten.

## Claims

1. A process for the preparation of a granular composition comprising solidified melt granules comprising a non-steroidal anti-inflammatory drug (NSAID) as a continuous phase wherein the NSAID is ibuprofen, flurbiprofen, ketoprofen, naproxen or an enantiomer or a salt thereof, which process comprising the steps of:
(a) melt-extruding the NSAID;
(b) forming a homogeneous extrudate of the NSAID;
(c) cooling the extrudate; and
(d) comminuting the cooled extrudate to form granules;
wherein in step (a) the NSAID is fully melted and in step (b) the extrudate is formed into two or more ribbons having a depth of 10 mm or less and which solidify in 5 minutes or less wherein the NSAID is heated to a temperature of 5 - 20°C above its melting point wherein a disintegrant is melt extruded with the NSAID, wherein the disintegrant is selected from croscarmellose sodium and/or sodium starch glycolate and wherein the NSAID forms 70 - 95% w/w of the melt granules.

2. A process according to claim 1, wherein the NSAID has a melting point of from 30 to 300°C.

3. A process according to any one of the preceding claims wherein step (c) takes place on a cooling means selected from a cooling belt and a cooling drum.

4. A process according to any one of the preceding claims wherein the width of each ribbon of molten extrudate is greater that the depth of the ribbon.

5. A process according to any one of the preceding claims wherein the ribbons of molten extrudate solidify in 1 minute or less.

6. A process according to any one of the preceding claims wherein each ribbon of molten extrudate has a depth of up to 6 mm.

7. A process according to any one of the preceding claims wherein each ribbon of molten extrudate has a depth of 0.5-4mm.

8. A process according to any one of the preceding claims wherein each ribbon of molten extrudate has a depth of 1-3 mm.

9. A process according to any one of the preceding claims wherein three or more ribbons of molten extrudate are formed.

10. A process according to any one of the preceding claims wherein the temperature difference between said molten extrudate and the cooling means as it comes into contact with cooling means is at least 50°C.

11. A process according to claim 10 wherein the NSAID is racemic ibuprofen or S (+)-ibuprofen.

12. A process according to any one of the preceding claims wherein step (a) is carried out in an extruder having at least one screw shaft provided with means arranged to generate heat within the NSAID.

13. A process according to any one of the preceding claims in which a twin screw extruder is employed.

14. A process according to any one of the preceding claims wherein the extruder comprises an extruder barrel heated to a temperature of in the range of 80 -130°C.

15. A process according to any one of the preceding claims wherein the NSAID is fully molten as it exits the extruder.

16. A process according to any one of the preceding claims wherein the solid composition collected is milled through a screen having a round hole size of less than 2mm.

## Patentansprüche

1. Verfahren zur Herstellung einer körnigen Zusammensetzung, umfassend verfestigtes Schmelzgranulat, umfassend ein nicht-steroidales Antirheumatikum (NSAR) als kontinuierliche Phase, wobei das NSAR Ibuprofen, Flurbiprofen, Ketoprofen, Naproxen oder ein Enantiomer oder ein Salz davon ist, wobei das Verfahren die folgenden Schritte umfasst:
(a) Schmelzextrusion des NSAR;
(b) Bilden eines homogenen Extrudats des NSAR;
(c) Abkühlen des Extrudats; und
(d) Zerkleinern des abgekühlten Extrudats zur Bildung von Granulat;
wobei in Schritt (a) das NSAR vollständig geschmolzen wird und in Schritt (b) das Extrudat zu zwei oder mehr Bändern geformt wird, die eine Tiefe von 10 mm oder weniger aufweisen und sich in 5 Minuten oder weniger verfestigen, wobei das NSAR auf eine Temperatur von 5-20°C über seinem Schmelzpunkt erhitzt wird, wobei ein Sprengmittel mit dem NSAR Schmelzextrusion unterworfen wird, wobei das Sprengmittel unter Croscarmellose-Natrium und/oder Natriumstärkeglykolat ausgewählt ist und wobei das NSAR 70-95% w/w des Schmelzgranulats bildet.

2. Verfahren nach Anspruch 1, wobei das NSAR einen Schmelzpunkt von 30 bis 300°C aufweist.

3. Verfahren nach einem der vorhergehenden Ansprüche, wobei Schritt (c) auf einem Kühlmittel erfolgt, das unter einem Kühlband und einer Kühltrommel ausgewählt ist.

4. Verfahren nach einem der vorhergehenden Ansprüche, wobei die Breite jedes Bands aus geschmolzenem Extrudat größer als die Tiefe des Bands ist.

5. Verfahren nach einem der vorhergehenden Ansprüche, wobei sich die Bänder aus geschmolzenem Extrudat in 1 Minute oder weniger verfestigen.

6. Verfahren nach einem der vorhergehenden Ansprüche, wobei jedes Band aus geschmolzenem Extrudat eine Tiefe von bis zu 6 mm aufweist.

7. Verfahren nach einem der vorhergehenden Ansprüche, wobei jedes Band aus geschmolzenem Extrudat eine Tiefe von 0,5-4 mm aufweist.

8. Verfahren nach einem der vorhergehenden Ansprüche, wobei jedes Band aus geschmolzenem Extrudat eine Tiefe von 1-3 mm aufweist.

9. Verfahren nach einem der vorhergehenden Ansprüche, wobei drei oder mehr Bänder aus geschmolzenem Extrudat gebildet werden.

10. Verfahren nach einem der vorhergehenden Ansprüche, wobei der Temperaturunterschied zwischen dem geschmolzenen Extrudat und dem Kühlmittel, wenn es mit dem Kühlmittel in Kontakt kommt, mindestens 50°C beträgt.

11. Verfahren nach Anspruch 10, wobei das NSAR racemisches Ibuprofen oder S(+)-Ibuprofen ist.

12. Verfahren nach einem der vorhergehenden Ansprüche, wobei Schritt (a) in einem Extruder mit mindestens einer Schneckenwelle durchgeführt wird, die Mittel aufweist, die zur Erzeugung von Wärme im NSAR angeordnet sind.

13. Verfahren nach einem der vorhergehenden Ansprüche, in dem ein Doppelschneckenextruder verwendet wird.

14. Verfahren nach einem der vorhergehenden Ansprüche, wobei der Extruder einen Extruderzylinder umfasst, der auf eine Temperatur im Bereich von 80-130°C erwärmt wird.

15. Verfahren nach einem der vorhergehenden Ansprüche, wobei das NSAR vollständig geschmolzen ist, wenn es den Extruder verlässt.

16. Verfahren nach einem der vorhergehenden Ansprüche, wobei die feste gesammelte Zusammensetzung durch ein Sieb mit einer Rundlochgröße von weniger als 2 mm gemahlen wird.

## Revendications

1. Procédé de préparation d'une composition granulaire comprenant des granules de matière fondue solidifiée comprenant un anti-inflammatoire non stéroïdien (AINS) en tant que phase continue, où l'AINS est l'ibuprofène, le flurbiprofène, le kétoprofène, le naproxène ou un énantiomère ou sel de ceux-ci, ledit procédé comprenant les étapes consistant à :
(a) extruder à l'état fondu l'AINS ;
(b) former un extrudat homogène de l'AINS ;
(c) refroidir l'extrudat ; et
(d) broyer l'extrudat refroidi pour former des granules ;
où dans l'étape (a) l'AINS est entièrement fondu, et dans l'étape (b), l'extrudat est façonné pour former deux rubans ou plus d'une profondeur de 10 mm ou moins, et qui se solidifient en 5 minutes ou moins, où l'AINS est chauffé jusqu'à une température de 5 à 20 °C au-dessus de son point de fusion, où un agent délitant est extrudé à l'état fondu avec l'AINS, où l'agent délitant est choisi parmi le croscarmellose sodium et/ou le glycolate d'amidon sodium et où l'AINS forme 70 à 95 % en masse des granules de matière fondue.

2. Procédé selon la revendication 1, où l'AINS présente un point de fusion compris entre 30 et 300 °C.

3. Procédé selon l'une quelconque des revendications précédentes, où l'étape (c) se déroule sur un moyen de refroidissement choisi parmi un tapis de refroidissement et un tambour de refroidissement.

4. Procédé selon l'une quelconque des revendications précédentes, où la largeur de chaque ruban d'extrudat fondu est supérieure à la profondeur du ruban.

5. Procédé selon l'une quelconque des revendications précédentes, où les rubans d'extrudat fondu se solidifient en 1 minute ou moins.

6. Procédé selon l'une quelconque des revendications précédentes, où chaque ruban d'extrudat fondu présente une profondeur pouvant atteindre 6 mm.

7. Procédé selon l'une quelconque des revendications précédentes, où chaque ruban d'extrudat fondu présente une profondeur de 0,5 à 4 mm.

8. Procédé selon l'une quelconque des revendications précédentes, où chaque ruban d'extrudat fondu présente une profondeur de 1 à 3 mm.

9. Procédé selon l'une quelconque des revendications précédentes, où trois rubans d'extrudat fondu ou plus sont formés.

10. Procédé selon l'une quelconque des revendications précédentes, où la différence de température entre ledit extrudat fondu et le moyen de refroidissement lorsque le premier entre en contact avec le second est d'au moins 50 °C.

11. Procédé selon la revendication 10, où l'AINS est l'ibuprofène racémique ou le S(+)-ibuprofène.

12. Procédé selon l'une quelconque des revendications précédentes, où l'étape (a) est mise en oeuvre dans une extrudeuse ayant au moins un axe de vissage pourvu de moyens disposés de sorte à générer de la chaleur à l'intérieur de l'AINS.

13. Procédé selon l'une quelconque des revendications précédentes, dans lequel une extrudeuse à double vis est employée.

14. Procédé selon l'une quelconque des revendications précédentes, où l'extrudeuse comprend un corps cylindrique d'extrudeuse chauffé à une température comprise entre 80 et 130 °C.

15. Procédé selon l'une quelconque des revendications précédentes, où l'AINS est entièrement fondu lorsqu'il sort de l'extrudeuse.

16. Procédé selon l'une quelconque des revendications précédentes, où la composition solide collectée est broyée à travers un crible présentant une taille d'orifices ronds de moins de 2 mm.
